# EUROPEAN PATENT SPECIFICATION

(11) **EP 0 746 356 B1**
(45) Date of publication and mention of the grant of the patent: **17.05.2000**
(21) Application number: 92921191.0
(22) Date of filing: 18.09.1992
(51) Int. Cl.: A61M 5/155

(54) **GAS PRESSURE DRIVEN INFUSION SYSTEM BY HYDROGEL ELECTROLYSIS**
DURCH MITTELS ELEKTROLYSE IN HYDROGEL ERZEUGTEN GASDRUCK ANGETRIEBENES INFUSIONSSYSTEM
DISPOSITIF DE PERFUSION UTILISANT LA PRESSION D'UN GAZ PAR ELECTROLYSE EN HYDROGELS

(30) Priority: 24.10.1991 US 783634
(43) Date of publication of application: 11.12.1996
(73) Proprietor: INSUTECH, INCORPORATED, Salt Lake City, UT 84108 (US)
(72) Inventor: BAE, You, Han, Salt Lake City, UT 84108 (US); KWON, Ick, Chan, Salt Lake City, UT 84108 (US)
(74) Representative: Gilmour, David Cedric Franklyn
(86) International application number: PCT/US92/08046
(87) International publication number: WO 93/07920

(56) References cited:
- EP-A- 0 385 916
- WO-A-91/00753
- FR-A- 2 195 461
- US-A- 2 036 739
- US-A- 2 445 477
- US-A- 3 022 785
- US-A- 3 115 280
- US-A- 5 002 005
- US-A- 5 116 312
- US-A- 5 125 894

## Description

### Background of the Invention

This invention relates to a system for the administration of drugs and/or biologically active materials by continuous intervenous infusion, utilizing gas pressure produced by the hydrogel electrolysis. More particularly, this invention relates to a delivery device which utilizes gas pressure from free oxygen and hydrogen derived from the electrolysis of water in hydrogels. The gas pressure forces the infusion of the drugs and/or biologically active materials through appropriate means into the body. The rate of electrolysis which produces the gases (oxygen and hydrogen) is controlled by an electric current. This current is supplied by a battery or is activated and controlled by an electronic timer or a biomedical control system which reacts to stimuli related to bodily functions, such as temperature, pH, muscle contractions, electroencephalography, or electrocardiography, and/or a combination of the above.

### Description of Prior Art

There have been many approaches to meet the problems of regulating the delivery of drugs and/or biologically active materials (hereinafter collectively referred to as "drugs") in the place and at the proper dose to achieve the desired regulatory effect. Some of these systems depend on the utilization of physical or chemical stimuli which are a result of changes in the biological systems. These changes are usually of an external nature to the drug delivery system. These mechanisms respond to such stimuli or signals which include protein binding, hydrogel expanding or swelling, polymer erosion, membrane reorganization, solubility change, energy conversion, supply of activation energy for permeation, physical property changes of the materials that comprise the system, or phase transition phenomena, and the like. Examples are presented in J. Heller, *Chemically self-regulated drug delivery systems*, J. Control. Rel., 8, 111-125 (1988) and J. Kost (ed.), *Pulsed and Self-Regulated Drug Delivery* CRC Press. Inc. Boca Raton, Florida, 1990.

Other delivery systems currently available utilize gravity flow and other electrically driven mechanical pumps (peristaltic or syringe pumps) attached to syringes or intervenous tubing which infuse the drugs into the body. In addition, elastomeric balloons can also be utilized as the contraction force E. Bruerar et al., *Continuous sc infusion of narcotics using a portable disposable device in patients with advanced cancer*, Cancer Treatment Report, **71, 635-637** (1987) for the portable infusion systems. These systems require large, complicated supports along with electronic or mechanical pumps which restrict their portability for ambulatory patients in hospitals or at home. Moreover, these systems have constant infusion rates which cannot be regulated in a time-released pattern required by some drugs, such as anti-cancer agents.

European Patent specification 0 385 916, Gross et al., published Sep. 5, 1990 describes a container including a displaceable partition to define a first expansible-contractible chamber on one side for receiving the liquid to be dispensed, and a second expansible-contractible chamber on the opposite side for receiving an electrolytic cell. The electrolytic cell has electrodes and an electrolyte capable of generating, upon the energization of the electrodes, a gas under pressure to displace the displaceable partition and to force the liquid out from the first chamber in accordance with the rate of energization of the electrodes. The electrolyte used is stated to include saline solution, other polar solutions or liquid gels, generating hydrogen, oxygen, nitrogen or carbon dioxide.

### Objects and Brief Summary of the Invention

It is an object of the present invention to provide a delivery system which does not use mechanical motors or gears as a propulsion means for the infusion of drugs.

It is also an object of this invention to produce a gas generator which utilizes water swollen hydrogels that can be electrolyzed into gases (oxygen and hydrogen) to produce the propulsion means for the infusion of drugs.

According to the present invention there is provided a gas driven drug delivery device for dispensing a liquid drug at a predetermined rate, comprising:
(a) a gas generation unit for producing oxygen and hydrogen gases by the electrolysis of water comprising a housing having affixed therein a hydrogel, electrodes inserted into said hydrogel and extending outwardly of said housing and means attached to said electrodes outwardly of said housing for applying an electric current to said electrodes;
(b) a non-expandable fluid reservoir in sealed fluid communication with said gas generation unit containing a drug solution and configured for receiving oxygen and hydrogen produced at said electrodes under pressure, said reservoir having an outlet passageway; and
(c) delivery means communicating with said outlet passageway in said reservoir for receiving displaced drug solution from said reservoir in response to the pressure exerted by oxygen and hydrogen generated in said gas generation unit entering said reservoir and displacing drug solution from said reservoir through said outlet passageway and directing said solution into the body of a patient, said device being characterized by said hydrogel comprising an electroconductive solid water swollen negatively charged polymeric network possessing strength and rigidity, wherein said negatively charged polymeric network comprises an acidic polymer composed of synthetic, semi-synthetic, or natural monomers that contain carboxylic or sulfonic acid groups.

The present invention thus provides a water swollen hydrogel system, which allows electric current to flow through the hydrogel resulting in electrolysis of water, to produce gases (oxygen and hydrogen).

The present invention also produces a simple and disposable means of infusion in which the infusion rate for the drugs can be controlled by an electric current.

The device of the present invention enables the provision of a compact infusion unit which is controlled by electrical current supplied by lightweight, leak proof batteries and regulated by an electronic timer or other biomedical control means.

Furthermore, the invention enables construction of a simple, disposable propulsion means which is capable of delivering drugs in a timed pattern that can be attached or adopted to already existing infusion vehicles.

Still further the present invention enables the creation of a disposable syringe system which is capable of delivery drugs in a timed pattern and additionally the creation of a simple and disposable infusion unit which can be employed in portable programmable infusion systems.

Also, the present invention enables the provision of a delivery system for drugs which is activated by a biomedical control system that reacts to stimuli related to bodily functions, such as temperature, pH, muscle contractions, electroencephalography, or electrocardiography and/or combinations of the above.

The device of the invention may be implemented by means of a simple infusion system which is composed of either a single or multiple units. In a preferred embodiment of the invention the single unit system is composed of a rigid container, in the shape of a chamber formed so that a piston will snugly engage the interior walls of said chamber and slides in response to the pressure of the expanding electrolytic gases to expel drug solution from said drug solution reservoir. This single infusion system can be separated into two spaces. The first space contains the water swollen hydrogel and is preferably divided from the second space, which contains the drug solution, by a rigid member that is fitted to the sides of the first space to act as a piston. This piston displaces the drug from the second compartment when the gas pressure is produced by the electrolysis of the water swollen hydrogel. The water swollen hydrogel is capable of conducting electrical direct current. Additionally, this first space contains an anode and a cathode that produce oxygen and hydrogen (gas) when an electric current supplied by a power source, such as a battery, which is regulated by or attached to an external control unit that is activated by an electronic timer or biomedical control unit, is applied. This biomedical control unit reacts to changes in bodily functions such as, temperature, pH, muscle contractions, electroencephalography, or electrocardiography, and/or any of the above in combination by producing an electric current that varies in intensity according to the strength of the stimuli. This current intensity is controlled either by voltage or by variation in the current density. This control unit is attached by electrical wires to the anode and cathode. The anode and cathode can either pass into the hydrogel through passageways in the walls of the container or pass into the hydrogel through a gas impermeable cap placed at the opening of the container. The passageways, in the rigid container, for these electrodes are sealed so that no liquid or gas can pass through. The second compartment has an opening which is incorporated into a syringe needle or is attached to tubing that carries the drug to a catheter or a hypodermic needle implanted in the body. This compartment is filled with a drug solution which is to be administered intervenously to the patient. The piston between the two compartments moves in response to the pressure of the gas formed by the electrolysis of the water swollen hydrogel, thereby forcing the drug through the opening into the syringe or tubing. Another embodiment of this single unit device does not have a piston but, permits expansion of gases (oxygen and hydrogen) to act directly on the drug solution forcing it through the syringe. The housing for this embodiment can be of non-expandable impermeable material which can be rigid or flexible. Depending on what type of delivery is desired for a particular drug, the electrical current can be precisely controlled in such a way that the drug can be timed or constant or a combination of both and the pressure exerted by the gases (oxygen and hydrogen) can be pulsating or constant or a combination of both. This single unit system can also be constructed so that the gas generator can be attached, such as by threaded or clamping means rendered gas tight by an appropriate gasket (such as an O-ring) to the compartment containing the drug. This construction provides a method of reusing the gas generator system. The multiple unit system is composed of a rigid container (gas generator) which contains a water swollen hydrogel capable of conducting electrical currents. Electrodes pass through the top of the rigid container (gas generator) into the water swollen hydrogel and are attached to batteries, the electric of which is regulated by a control unit that is activated by an electronic timer or a biomedical control unit which is responsive to changes in bodily functions such as, temperature, pH, muscle contractions, electroencephalography, or electrocardiography, and/or combinations of the above. The electrodes are sealed so that no liquid or gas can pass through the holes in the container. This rigid container (gas generator) has an opening that is attached, by way of flexible tubing, to a reservoir filled with the drug to be administered to the patient. This reservoir has a second passageway that is attached to a catheter or other means of infusion into the body. The reservoir can be formed from materials that are flexible but not expandable. When the water swollen hydrogel in the rigid container (gas generator) is subjected to the electrical currents, gases (oxygen and hydrogen) are formed. These gases exert pressure through the tubing on the drug in the reservoir, thereby forcing the contents of the reservoir through the second opening into the body.

### Brief Description of the Drawings

Fig. 1 is a longitudinal cross sectional view showing a two-piece infusion unit where the reservoir is filled with the drug.
Fig. 2 is a view of the unit of Fig. 1 which illustrates the operation of a two-piece infusion unit when an electric current has been applied to generate the gases (oxygen and hydrogen) which in turn forces the drug through an opening of the reservoir for delivery into the body.
Fig. 3 shows in longitudinal cross section a one-piece infusion syringe unit without a piston where the pressure from the generated gases (oxygen and hydrogen) exerts pressure directly on the a drug solution, displacing the solution and forcing the displaced solution from the syringe.
Fig. 4 shows in longitudinal cross section a one-piece infusion syringe where the drug is separated from the water swollen hydrogel by a piston which is driven against the drug solution when the production of gases (oxygen and hydrogen) occurs. The electrodes are connected through the side of the syringe.
Fig. 5 illustrates in longitudinal cross section another embodiment of a one-piece infusion syringe with a piston wherein the electrodes are connected through the top of the syringe.
Fig. 6 shows in longitudinal cross section an infusion syringe which has the electrodes in a separate top unit fitted to the barrel of the syringe which contains a piston. The top unit containing the electrodes is a attached to the barrel of the syringe and is made leak proof by sealing means such as an, O-ring or gasket. The gas generation unit is threaded onto the barrel.
Fig. 7 illustrates, also in longitudinal cross section, a gas generator portion wherein the electrodes contacting the water swollen hydrogel pass through the sidewalls of the housing. The top is sealed with an impermeable plug that does not allow the gases to escape into the atmosphere.
Fig. 8 shows a gas generator similar to Fig. 7 wherein the hydrogel acts as a stopper without an additional sealing component. Fig. 9 illustrates a gas generator similar to Fig. 7 where the electrodes pass through the sealing top into the hydrogel which is slightly separated from the sealing top.
Figs. 10a-d demonstrate methods for fabricating a hydrogel for a gas generator which is rigid and impermeable to the gases produced.
Fig. 11 illustrates a three dimensional sketch of the self-supporting hydrogel used in Figures 7 and 8.

### Detailed Descriptions of the Drawings

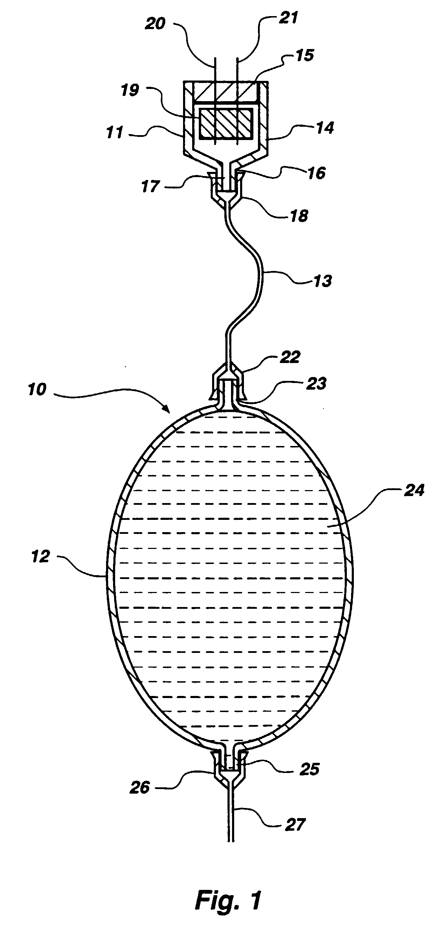

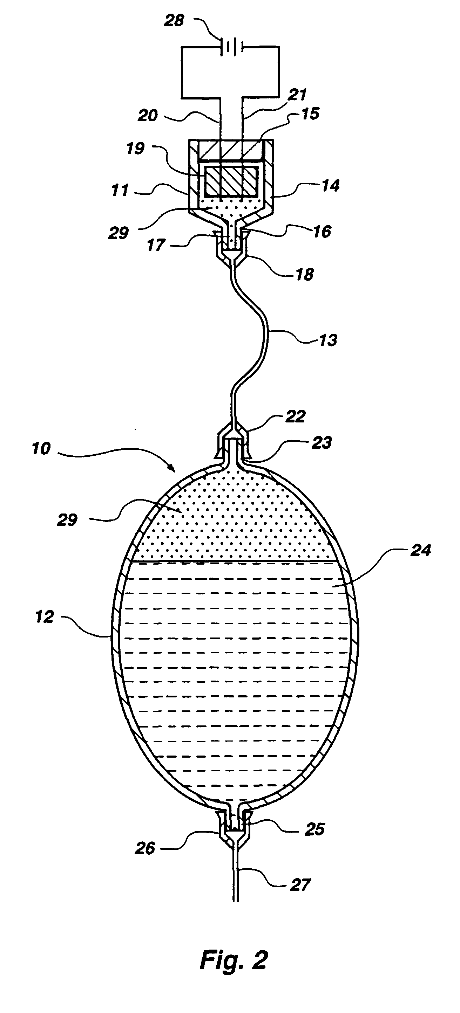

Figure 1. shows a two-piece system 10 for drug delivery which is composed of a gas generation unit 11 that is attached to a reservoir 12 by way of tubing 13. The gas generation unit 11 consists of a rigid housing 14 that is sealed on top by a sealing element 15 and is narrowed at the bottom into a projection 16 with an opening 17. The projection 16 is fitted with a connecting joint 17 which prevents the escape of gases (oxygen and hydrogen) into the atmosphere. Tubing 13 is attached to the connecting joint 17. Contained within housing 14 is a water swollen hydrogel 19. Electrodes 20 and 21 are inserted into hydrogel 19 and protrude from the hydrogel through the sealed top 15 and are attached to wires connected to a control unit, the current of which is supplied by batteries (not shown). The tubing 13 that is attached to the gas generation unit 11 is connected to a connecting joint 22 fitted to a hollow upper protrusion 23 on a reservoir 12 that contains the liquid drug formulation 24. The reservoir 12 has an additional lower hollow protrusion 25 engaged through a connecting joint 26 to tubing 27 that leads to a catheter or hypodermic needle (not shown), which is implanted into the body. Figure 2 illustrates the operation of the same device described in Figure 1 with an applied electric current from a biomedical control unit 28. Under the applied current to electrodes 20 and 21 the hydrolysis of the water in the hydrogel 19 occurs and gases (oxygen and hydrogen) 29 (shown as dots in the figures) are produced which expand and pass through the tubing 13 that connects the gas generator 11 with the reservoir 12. The drug solution 24 in the reservoir 12 is displaced by these gases 29 as they expand into the reservoir (oxygen and hydrogen). The drug solution 24 is then forced through the tubing 27 for delivery into the body.

The gas generator housing (11) is constructed of rigid materials such as glass, metals, ceramics, and/or plastics such as polyethylene, polypropylene, polycarbonates, polystyrene, or other plastics that are rigid and impermeable to gases. The sealing element (15) is made from materials that are self-sealing and sufficiently compressible to form a tight seal against the rigid walls of the gas generator. Examples of these materials would be a semi-flexible rubber such as soft silicone rubber, neoprene, etc. The electrodes (20 and 21) are composed of non-sacrificial materials which conduct electric current and are not decomposed by the electrolysis reaction, for example gold, silver, copper, platinum and/or any of the foregoing metal coated electrodes. The tubing (13) is composed of flexible, non-porous material such as plasticized polyvinylchloride, polyurethane, polyethylene, polypropylene, silicone rubber, etc. The reservoir (12) is composed of non-expandable and impermeable materials that can be flexible or non-flexible such as glass, metals, ceramics, and/or plastics such as polyethylene, polypropylene, polycarbonates, polystyrene, polyvinylchloride or other plastics. The hydrogel (19) can be made of crosslinked polybases or polyacids, uncharged polymers, and polymers having linear polyelectrolytes as an additional component. The above polymers can be mixed with plasticizers that do not hinder the flow of electric current to the water swollen hydrogels. For hydrogels that have polyelectrolyte properties pure water can be used as the swelling agent.

The polyelectrolyte hydrogels can be formed from monomeric polybases and polyacids which are polymerized or crosslinked forming networks containing negatively charged ions. The acidic polymer networks are composed of synthetic, semi-synthetic, or natural monomers that contain carboxyl acid groups and/or sulfonic acid groups, singly or in combination. Examples of the synthetic acid monomers that can form the polymer are; styrene sulfonic acid, 2-acrylamido-2-methylpropane sulfonic acid, acrylic acid, methacrylic acid, allylacetic acid, maleic acid, for example. The semi-synthetic polymer networks can be composed of acid group containing polymerized natural substances that will absorb water, such as dextrin sulfate gel. The natural polymer network comprise, for example, protein gels, heperin gels, or a combination of both. See **J.D. Andrade** (Ed.), Hydrogels for Medical and Related Applications, *ACS Symposium Series No. 31*, American Chemical Society, Washington D.C., 1976; and **P. Molyneux, Water-Soluble Synthetic Polymers**, *Vol. I and II*, CRC Press, Inc., Boca Raton, Florida, 1984; and **N.A. Peppas**, Hydrogels in Medicine and Pharmacy, *Vol. I, Ii, and III*, CRC Press, Inc., Boca Raton, Florida.

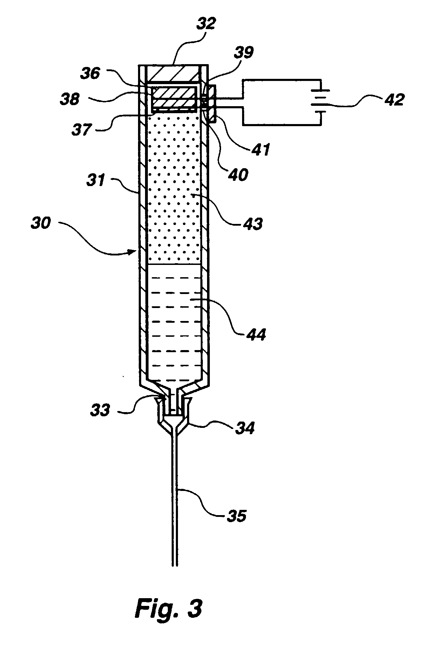

Figure 3 shows a syringe device 30 having a unitary, non-expandable container 31 sealed at the top by a sealing element 32 and tapered at the bottom ending in a hollow protrusion 33. A connection joint 34 is friction fitted about protrusion 33 and holds a needle or line 35. Secured in the upper portion of a unitary, non-expandable container 31 is hydrogel 36 into which is inserted electrodes 37 and 38. The electrodes 37 and 38 are inserted directly into the hydrogel 36 through holes 39 and 40 drilled in the wall of the container. The top of the container or barrel is plugged and sealed with materials 32 that are impermeable to the gases produced by the electrolysis. Sealant 41 is applied around the electrodes outside the barrel wall to cover holes 39 and 40 so as to prevent any possible leakage of gases (oxygen and hydrogen) or liquid formulation. A biomedical control unit or electronic timer 42 provides an applied electric current to electrodes 37 and 38 to produce hydrolysis. As hydrolysis of the water in the hydrogel 36 occurs, gases (oxygen and hydrogen) 43 (shown as dots) are produced which expand and exert pressure on the drug solution 44 in the unitary, non-expandable container 31. The drug solution 44 is displaced by the expanding gases 43 and the drug solution 44 is then forced through the needle or tubing 35 for delivery into the body.

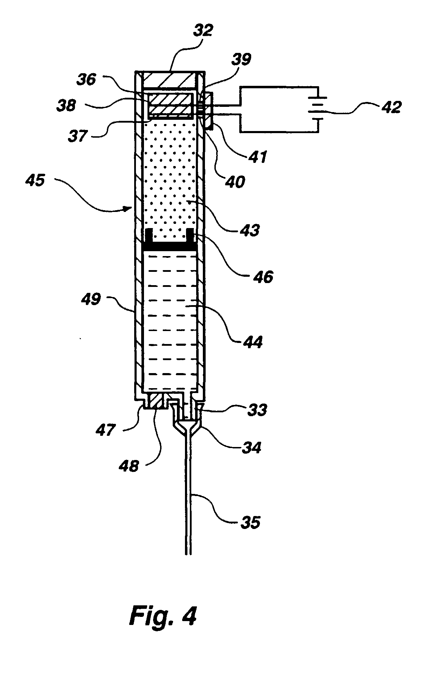

Figure 4 illustrates a device 45 similar to that shown in Figure 3 wherein comparable elements are identified by the same numerals as in Figure 3. However, the device 45 contains a piston 46 frictionally fitted into the rigid barrel 49 of the syringe. Piston 46 separates the gases 43 that are produced by electrolysis (oxygen and hydrogen) of the water in hydrogel 36 from the liquid phase drug solution 44. The piston 46 moves in response to the pressure of the gases produced by electrolysis occurring in the hydrogel. The rigid barrel 49, which is formed from part of a syringe, also has an opening (filling port) 47 that is used for filling the space below the piston 46 with a drug solution 44. This opening is stopped with a self-sealing material 48 that will seal itself after being punctured with a needle or hollow wire used for inserting the drug solution 44. This filling port 47 allows the drug solution 44 to be introduced into the syringe device 45 after the hydrogel 36 and electrodes 37 and 38 have been placed in the top of the barrel 49. Any material which has self-sealing ability after puncturing by sharp needle or wire can be used for sealing the filling port 47. The piston 46 in this embodiment of the device prevents the gases from coming in contact with the drug solution thereby preventing contamination of the drug with gases or the injection of the gases into the body. The barrel 49 of the device 45 should be rigid and cylindrical in shape with a uniform diameter. Besides the pressure of the gases, the friction between the piston 46 and the inner surface of the cylinder wall of barrel 49 is an additional factor that influences the infusion rate.

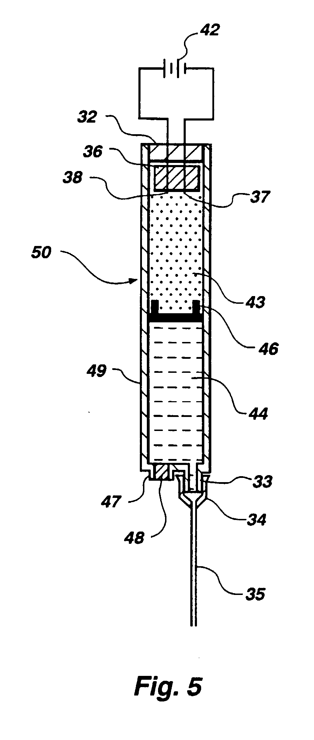

Figure 5 illustrates another embodiment of the device shown in Figure 4. This device 50, instead of having electrodes 37 and 38 being inserted into the hydrogel 36 through holes in the side of the cylinder, has electrodes inserted through the plug 32 at the top of the barrel 49 into the hydrogel. This configuration eliminates the additional need of sealing the holes in the side of the cylinder for the electrodes of Figure 4.

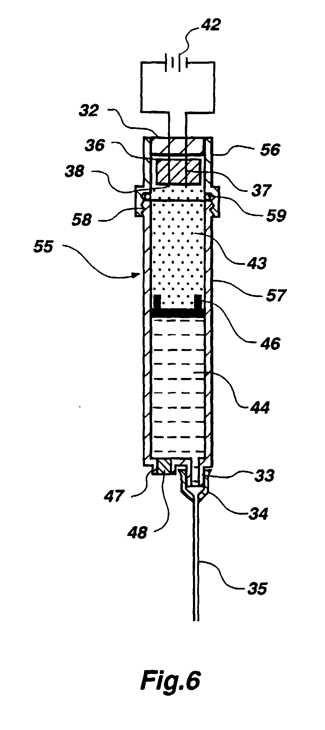

Figure 6 illustrates a device 55 composed of two separate parts, i.e. a gas generator portion 56 that contains electrodes 37 and 38 inserted through a sealed top 32 into a hydrogel 36, and a cylindrical drug container portion 57 with a piston 46 frictionally fitted into the interior to prevent gas leakage providing a space that can be filled with a drug solution 44 and into which electrolysis gases 43 can expand. The gas generator portion 56 can be coupled with the cylindrical container portion 57 by a threaded means 58, or any other coupling systems that would seal the gas generator portion 56 to the cylinder container portion 57. For example, flanges around the lower and upper ends of generator portion 56 and container portion 57 respectively separated by a gasket and clamped together are one alternative. As shown in Fig. 6, an O-ring gasket 59 fitted between the two parts 56 and 57 prevents gas leakage. In this embodiment the gas generator 56 can be recharged with fresh hydrogel 36 if needed and be reused.

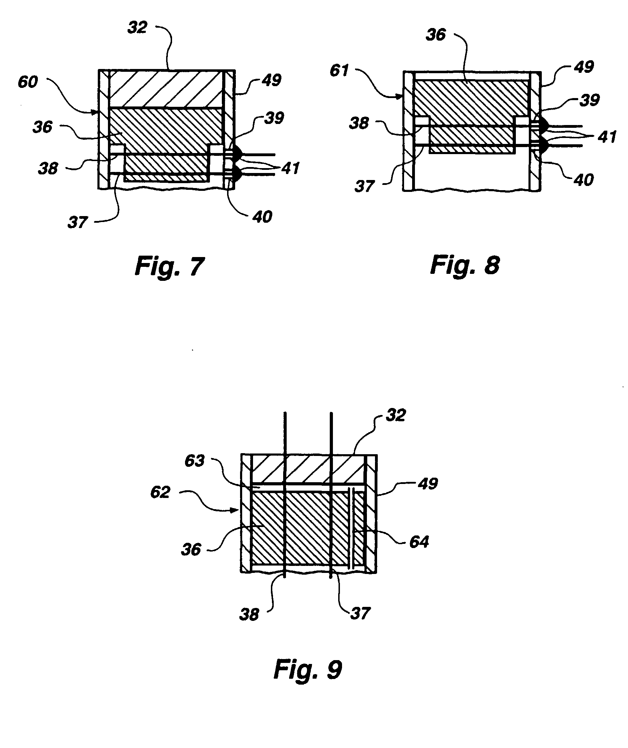

Figures 7-9 illustrate the various embodiments of gas generators which can be utilized when the mechanical properties of gels and/or electrodes are not strong enough to support the gel weight. Each of these systems use the gel's swelling power when hydrated to apply pressure on the cylinder walls thereby forming a rigid gel which supports its own weight and provides a gas tight seal.

Figure 7 shows a gas generator portion 60 composed of a sealing top 32, a self-supporting hydrogel 36 securely fitted into the cylinder top of the barrel 49 and in contact with both the sealing top 32 and inner walls of barrel 49. Electrodes 37 and 38 are inserted into the hydrogel 36 through barrel wall electrode holes 39 and 40, and sealant 41 surrounds the electrode wires and seals the electrode holes. In this embodiment the hydrogel may or may not form a gas tight seal with the cylinder walls 49.

Figure 8 illustrates a gas generator 61 similar to the generator of Figure 7 where the self-supporting hydrogel 36 is snugly fitted against the cylinder walls 49, forming a gas tight seal, eliminating the need for the sealing top. All other elements are the same as in Figure 7.

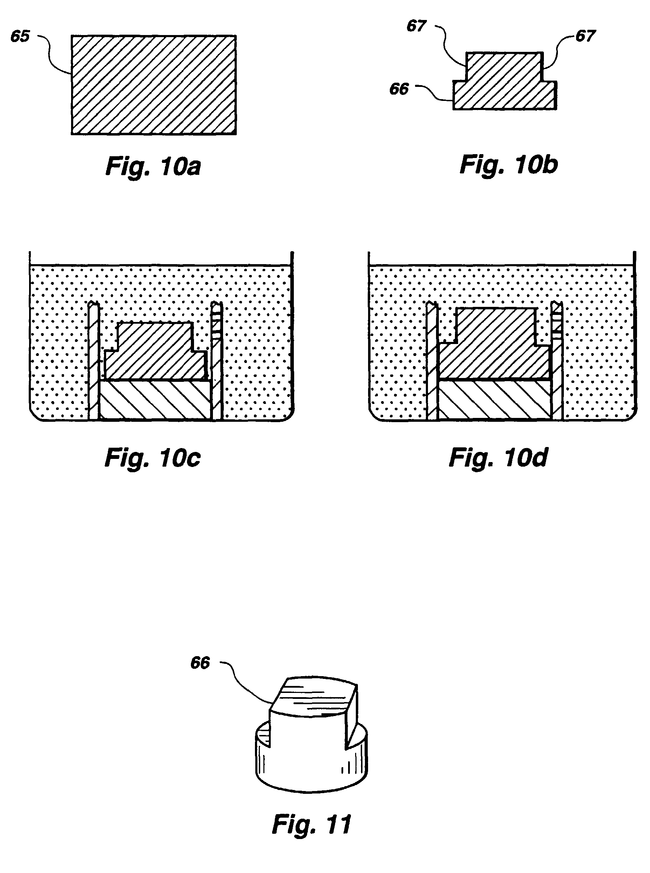

Figure 9 illustrates a gas generator device 62 where the electrodes 37 and 38 are inserted into the hydrogel 36 through the sealing top 32 thus eliminating the additional sealing step of sealing electrode holes in the side of the barrel 49. In addition, the hydrogel 36 may or may not form a gas tight seal with the container wall 49. A space 63 between the hydrogel 36 and the sealing top 32, along with one or more gas conduits 64 in the hydrogel itself are necessary in order for the gases that are formed in the upper portion of the hydrogel to pass below the hydrogel and properly exert pressure on a piston or drug solution. Figure 10 demonstrates the fabrication of a portion of a gas generator that contains the hydrogel fitted into the top of the generator before the electrodes are inserted into the hydrogel through the cylinder wall. This hydrogel is fitted into the cylinder, so as to be self-supporting and/or gas tight. Figure 10a depicts a cross section of a fully swollen hydrogel 65 cut in a cylindrical shape having a diameter which is larger than the diameter of a container into which it will be inserted. Figure 10b shows the hydrogel 66, in dehydrated or shrunken form, into which passages 67 have been cut to allow electrolysis gases emanating from a water swollen hydrogel under an applied current to be directed into a syringe or other appropriate expansion chamber. The dried hydrogel 66 depicted in Fig. 10b has been shrunk until its diameter is smaller than the barrel diameter of the container into which it will be inserted.

Figure 10c shows the placement of the hydrogel 66 from Figure 10b in the gas generator portion of a syringe or other container 31 adjacent a sealing top 32 and immersed in an aqueous solution optionally containing ions where the hydrogel does not have polyelectrolyte properties in a container for sufficient time for hydration and swelling. The wall of container 31 or 49 has electrode holes 39 and 40 for the subsequent insertion of electrodes following hydration of the hydrogel 66. Figure 10d is the same as Figure 10c but shows the hydrogel 66 in a swollen or hydrated state and snugly fitted into and in contact with the interior wall of container 31 or 49 thereby forming a seal with the wall that effectively supports the hydrogel and may also prevent the gases from escaping. The swollen hydrogel 66 is capable of supporting its own weight because of the tight fit with the interior surface of the wall of the container.

Figure 11 illustrates the three-dimensional shape of hydrogel 66 which is cut to make passageways 67 that provide a means of conducting the generated gases into a chamber that is provided for expansion of the gases.

The unitary, non-expandable container 31 is fabricated from materials that are impermeable to gases, for example polyethylene, polypropylene, etc. The rigid barrel 49 is manufactured from rigid materials such as glass, metals, ceramics, and/or plastics such as polyethylene, polypropylene, polycarbonates, polystyrene, or other plastics.

The pistons or plungers 46 are made of rigid materials that maintain a seal against the barrel walls, as the piston or plunger moves in response to the gas pressure exerted by the gas generator. Examples of these materials are; vulcanized rubber, ground glass , and plastics such as polyethylene, polypropylene, and polytetrafluoroethylene.

The sealing element 32 is manufactured from materials that are self-sealing and sufficiently compressible to form a tight seal against the rigid walls of the gas generator. Examples of these materials would be a semi-flexible rubber such as soft silicone rubber.

The electrodes (37 and 38) are composed of non-sacrificial materials which conduct electric current and are not decomposed by the electrolysis reaction, for example gold, silver, copper, platinum and/or any of the foregoing metal coated electrodes.

The sealant 41 is produced from materials that seal, preventing the leakage of gases, such as a silicone sealer.

The hydrogel 36 can be made of crosslinked polyacids, and polymers having linear polyelectrolytes as an additional component as illustrated above. The above polymers can be mixed with plasticizers that do not hinder the flow of electric current to the water swollen hydrogels. For hydrogels that have polyelectrolyte properties pure water can be used as the swelling agent.

### Detailed Description Preferred Embodiments of the Invention

As can be seen from the above description of the various embodiments shown in the drawings, the invention is directed to devices that deliver drug solutions through syringes or catheters into the patient. The drug is infused into the body by pressure produced through the electrolysis of water in a hydrogel. The gases (oxygen and hydrogen) produced by the electrolysis act as a motive force to produce pressure either directly on the drug solution or indirectly by moving a piston that produces force, at a constant rate, against the drug solution. As a result of this pressure, the drug is forced through a syringe or catheter into the patient's body.

When an electric current is applied to the water swollen hydrogel electrolysis of the water generates two hydrogen molecules (gas) and one oxygen molecule (gas) from two molecules of the entrained water molecules (liquid). The pressure produced by the generation of these gases is proportional to the electric current that is applied to the hydrogel. When the electrical current is applied to the swollen hydrogel, ions present in the gel phase can transfer electrons through the hydrogel allowing current to flow. The current causes the electrolysis of the surrounding water molecules in the hydrogel, resulting in generation of a hydrogen and oxygen gas mixture.

The chemical equation for the electrolysis of water entrained in a hydrogel is as follows:

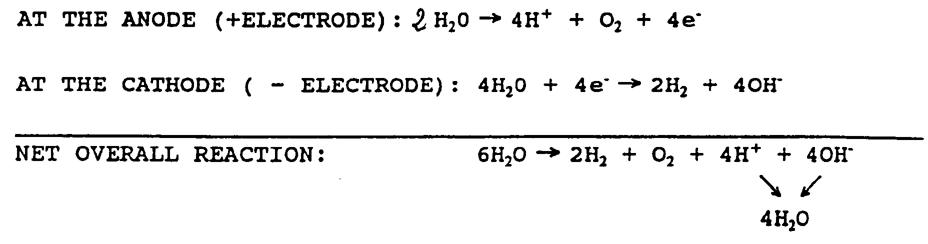

As a result, two moles of water (about 36 ml) will generate 3 moles of gas mixture (oxygen and hydrogen) which will have a volume of approximately 67.2 liters under standard conditions. Based upon the above measurements, the electrolysis of 26.7 mg water can displace 50 ml of drug solution. Moreover, the electrolysis of 5.34 mg water can replace 10 ml of liquid formulation. The electrolysis removes the above quantities of water from the hydrogel.

More particularly, the gas generator is composed of a housing, water swollen hydrogels, electrodes, and means to prevent gases from escaping The housing is constructed of rigid material such as glass, metals, ceramics, and/or plastics such as polyethylene, polypropylene, polycarbonates, polystyrene, or other plastics that would are rigid and impermeable to gases. The gas generator is constructed so it can be reused if desired. The hydrogels can be made of crosslinked polyacids, and polymers having linear polyelectrolytes as an additional component. The above polymers can be mixed with plasticisers that do not hinder the flow of electric current to the water swollen hydrogels. For hydrogels that have polyelectrolyte properties pure water can be used as the swelling agent. The electrodes should be composed of materials which conduct electric current and are not decomposed by the electrolysis reaction, for example gold, silver, copper, platinum and/or electrodes coated with the foregoing metals. An example of the sealing materials that can be used for the top of the gas generator is a flexible rubber such as soft silicone rubber.

A space should be left between the sealing top of the gas generator and the hydrogel that is fitted against the walls of the gas generator. The hydrogel is formed in such a way that passageways are present which allow free passage of the generated gases to the drug solution reservoir.

The pistons or plungers of the drug delivery device should be made of rigid materials that are sufficiently flexible to maintain a seal against the barrel walls, as the piston or plunger moves in response to the gas pressure exerted by the gas generator. Examples of these materials are; vulcanized rubber, ground glass , plastics such as polyethylene, polypropylene, and polytetraethylene fluoride.

All materials that are used in the fabrication of these drug delivery devices should be of biomedical grade and sufficiently sturdy to prevent damage from an accidental recombination of the gases (oxygen and hydrogen).

The delivery devices described above are particularly suitable for the delivery of drugs requiring pulsed delivery such as anti cancer drugs. These delivery devices are also useful for drugs requiring exact dosage in response to physical stimuli such as temperature, blood chemistry, rate and rhythm of heart and changes in electroactivity of the brain, more specifically antipyretics (acetylsalicylic acid, acetaminophen, etc.), anti- hyperglycemic agents (insulin etc.), agents for controlling cardiac erythema (atropine, digitalis etc.) and anticonvulsants ( hydantoin, barbiturates etc.), for example.

The following specific examples are directed to the use of water in the drug delivery devices, however, it is intended the devices can be used with any drug solution.

### Example 1

Preparation of Hydrogel: A random, crosslinked copolymer of 2-acrylamido-2-methylpropane sulfonic acid (AMPS) and n-butylmethacrylate (BMA) was synthesized in the following manner: Dry nitrogen gas was bubbled for 20 minutes through a mixture of AMPS and BMA (total 5.39 gram, 27/73 mole ratio), dimethylforamide (5 ml), N,N'-azobisisobutyronitrile (0.1 mole % of monomer), and ethylene glycol dimethacrylate (0.8 mole % of monomer). The above mixture was polymerized in a sealed glass tube (1 cm diameter and 10 cm length) at 60° C for 3 days. After polymerization, the polymer was removed from the glass tube by breaking the glass and was soaked in a water/acetone (50/50 v/v%) solvent mixture, the solvent was replaced daily for at least one week. The water/acetone solvent mixture in the resulting polymer was replaced with distilled water by gradually increasing the water content of the mixture until all of the acetone was removed from the polymer. The polymer was then kept in distilled water until used. The swelled hydrogel contains 70% by weight of water at a neutral pH and is slightly rigid.

### Example 2

Disposable polypropylene syringes of 3 ml capacity (0.85 cm inside diameter) were utilized in assembling a device similar to that shown in Figure 1. The plungers were removed from three syringes, leaving the hollow plastic barrels. The open end of each barrel was cut to give one barrel of 6 cm length and two barrels each having a length of 1.5 cm.

A gas generator unit was prepared from one of the 1.5 cm barrels. A fully swollen polyelectrolyte hydrogel synthesized in Example 1 was cut into a cylindrical piece of 0.8 cm diameter and 0.5 cm length and firmly inserted into one open end of one of the 1.5 long barrels. A disc shaped from soft silicone rubber (1 cm diameter and 0.5 cm height) was cut from a silicone sheet and inserted over the hydrogel as a sealing cap. Two platinum wire electrodes of 3 cm length were cut and inserted into the hydrogel through the silicone rubber seal. The distance between electrodes was 3 mm.

The open end of the second 1.5 cm long barrel was connected to the open end of the 6 cm long barrel by way of silicone tubing of 0.7 cm inside diameter, 1.5 mm wall thickness, and 2.5 cm length. The resulting single reservoir container, having a length of about 10 cm, was capped at the opposite or dispensing end of the 6 cm barrel unit and filled with water.

The gas generator and reservoir container assemblies were then interconnected at the narrowed or dispensing ends of the 1.5 cm barrels through a polyethylene tubing of 0.8 cm inside diameter and 11 cm length. The connections between the syringe barrels and tubing were made via two 18 G hypodermic needles. Finally the plastic cap at the end of the 6 cm barrel unit was replaced with a 23 G hypodermic needle.

### Example 3

When a fixed electric potential of 3 volts was applied at the platinum electrodes of the device described in Example 2, the measured current was 0.8 mA and the rate of displacement was 11.8 µl/min.

### Example 4

The relationship of infusion rate to electric current obtained with the device described in Example 2 was proportionally linear as shown in Table 1. The electric current was controlled by a potentiometer.

**Table 1**

| current (mA) | infusion rate (µl/min) |
|---|---|
| 0.77 | 10.2 |
| 0.8 | 11.8 |
| 0.85 | 11.8 |
| 1.05 | 15.2 |
| 1.1 | 14.4 |
| 1.15 | 16 |
| 1.2 | 16 |
| 1.4 | 18.5 |
| 3.4 | 48 |
| 3.5 | 49.2 |
| 7.5 | 108 |

### Example 5

A commercial 10 ml capacity glass syringe (1.55 cm inside barrel diameter, 9.5 cm barrel length) was dissembled. The glass plunger end (pip part) of 1.5 cm length was cut from the plunger rod and inserted into the barrel 2 cm from the open end to function as a piston. A fully swollen polyelectrolyte hydrogel synthesized in Example 1 was cut into a cylindrical piece of 1.1 cm diameter and 1 cm length and placed in the open end of the syringe above the piston. A piece of neoprene rubber ( 1.7 cm diameter and 0.5 cm height) was cut and shaped into a disc form and placed over the hydrogel to seal the open end of the syringe in a gas tight relationship. Two platinum wires of 3 cm length were inserted into the hydrogel through the neoprene rubber top as electrodes. The distance between electrodes was 0.5 cm.

The lower empty space (10 ml volume) was filled with distilled water. This device has the configuration described in Figure 5.

### Example 6

An electric potential of 3.50 volts was applied to the device described in example 5. The infusion rate and current are presented in Table 2.

| Time (min) | Infustion Rate (µ/min) | Current (mA) |
|---|---|---|
| 0 | 0 | |
| 5 | 65.8 | 4.5 |
| 10 | 48.6 | 2.8 |
| 15 | 41.5 | 2.7 |
| 20 | 37.9 | 2.5 |
| 25 | 36.1 | 2.3 |
| 30 | 36.1 | 2.3 |
| 35 | 36.1 | |
| 40 | 34.2 | 2.25 |
| 45 | 32.6 | 2.2 |
| 50 | 32.6 | 2.15 |
| 55 | 32.6 | |
| 60 | 32.6 | 2.15 |
| 70 | 31.3 | 2.05 |
| 75 | 32.6 | |
| 80 | 32.6 | |
| 90 | 29.9 | 2.0 |
| 100 | 33.5 | |
| 110 | 33.5 | 2.2 |
| 120 | 34.4 | 2.2 |
| 130 | 32.6 | 2.1 |
| 140 | 32.6 | 2.1 |
| 150 | 32.6 | 2.1 |
| 160 | 33.6 | |
| 170 | 32.6 | 2.1 |
| 180 | 31.8 | 2.08 |
| 190 | 32.6 | 2.05 |
| 200 | 31.8 | 2.1 |
| 210 | 30.8 | 2.08 |
| 220 | 31.8 | |
| 230 | 32.6 | 2.1 |
| 240 | 34.4 | 2.08 |
| 250 | 30.8 | 2.05 |
| 260 | 31.7 | |
| 270 | 31.6 | 2.05 |
| 280 | 30.7 | 2.05 |
| 290 | 30.8 | 2.08 |
| 295 | 32.4 | 2.05 |
| 300 | 0 | |

### Example 7

A disposable polypropylene syringe of 3 ml capacity (0.85 cm inside diameter) was utilized in assembling a device similar to that shown in Figure 4 but having a rubber stopper at the open end instead of a sealed top as described in Fig. 4. A rubber plunger tip was separated from the plunger rod and inserted, as a piston, into the barrel to give a space of 1.4 cm from the open end. Two holes of 0.6 mm diameter were drilled in the barrel for the electrodes. The locations of the holes were 1 cm and 0.7 cm apart respectively from the open end. The polymer hydrogel synthesized in Example 1 was cut into a disc 1.2 cm in diameter and 0.8 cm in length as shown in Fig. 10a. The hydrogel was then dried in air for 12 hours. The diameter of the partially dried hydrogel was smaller than 0.85cm. (the inside diameter of the barrel). This gel was then shaped as shown in Figures 10b and 11 by cutting off two pieces (0.4 cm from top and 0.15 cm from the side) to form gas passageways. The shaped gel was placed in the barrel and immersed in water in the manner shown in Figs. 10c and 10d (except no sealing plug was in the end of the barrel). This device was then kept in water for 12 hours. Prior to removal from the water the hydrogel was swollen and firmly inserted into the top of the barrel in the manner shown in Fig. 10d. A rubber stopper was inserted into the open end of the barrel over the hydrogel. Two platinum wire electrodes were inserted into the hydrogel through the holes in the barrel. A silicone sealant was applied around the electrodes and cured for 12 hours to prevent any leakage of gases (oxygen and hydrogen). The empty space below the piston was filled with distilled water. The final configuration was the same as Figure 4 except a rubber stopper was inserted into the open end of the barrel.

### Example 8

An electric potential of 3 volts was applied to the device described in example 7. The average current was 2.2 mA and pumping rate was 13.2 µl/min.

While the invention has been described and illustrated with reference to certain preferred embodiments thereof, those skilled in the art will appreciate that various modifications, changes, omissions, and substitutions can be made.

## Claims

1. A gas driven drug delivery device (10) for dispensing a liquid drug (24) at a predetermined rate, comprising:
a) a gas generation unit (11) for producing oxygen and hydrogen gases (29) by the electrolysis of water comprising a housing (14) having affixed therein a hydrogel (19), electrodes (20, 21) inserted into said hydrogel and extending outwardly of said housing and means attached to said electrodes outwardly of said housing for applying an electric current to said electrodes;
b) a non-expandable fluid reservoir (12) in sealed fluid communication (13) with said gas generation unit containing a drug solution (24) and configured for receiving oxygen and hydrogen (29) produced at said electrodes under pressure, said reservoir (12) having an outlet passageway (25); and
c) delivery means (27) communicating with said outlet passageway (25) in said reservoir (12) for receiving displaced drug solution (24) from said reservoir in response to the pressure exerted by oxygen and hydrogen (29) generated in said gas generation unit (11) entering said reservoir (12) and displacing drug solution from said reservoir (12) through said outlet passageway (25) and directing said solution (24) into the body of a patient, said device being characterized by said hydrogel (19) comprising an elastic electroconductive solid water swollen negatively charged polymeric network possessing strength and rigidity, wherein said negatively charged polymeric network comprises an acidic polymer composed of synthetic, semi-synthetic, or natural monomers that contain carboxylic or sulfonic acid groups.

2. A gas driven drug delivery device according to Claim 1 wherein the hydrogel is an acidic polymer network composed of monomers that contain sulfonic acid groups.

3. A gas driven drug delivery device according to Claim 2 wherein said monomers are members selected from the group consisting of styrene sulfonic acid and 2-acrylamido-2-methylpropane sulfonic acid.

4. A gas driven drug delivery device according to Claim 1 wherein the hydrogel is an acidic polymer network composed of monomers that contain carboxylic acid groups.

5. A gas driven drug delivery device according to Claim 4 wherein said monomers are members selected from the group consisting of acrylic acid, methacrylic acid and maleic acid.

6. A gas driven drug delivery device according to Claim 1 wherein the hydrogel is a natural polymer network composed of dextran sulfate gels, protein gels, heparin gels, and combinations thereof.

7. A gas driven drug delivery device according to Claim 1 wherein said negatively charged polymeric hydrogel is configured to allow hydrogen and oxygen gases produced at the electrodes to migrate out of said hydrogel and readily pass from any surface of said hydrogel in said gas generation unit to said gas compartment of said fluid container.

8. A gas driven drug delivery device according to Claim 7 wherein said hydrogel is in the form of a disk.

## Patentansprüche

1. Durch Gas angetriebene Arzneimittelverabreichungsvorrichtung (10) zum Abgeben eines flüssigen Arzneimittels (24) mit einer vorbestimmten Geschwindigkeit, welche folgendes umfaßt:
a) eine Gaserzeugungseinheit (11) zum Erzeugen von Sauerstoff- und Wasserstoffgasen (29) durch die Elektrolyse von Wasser, mit einem Gehäuse (14), welches darin angebracht ein Hydrogel (19) aufweist, Elektroden (20, 21), die in das Hydrogel eingesetzt sind und sich aus dem Gehäuse heraus erstrecken, und einer Vorrichtung, die an den Elektroden außerhalb des Gehäuses befestigt ist, zum Anlegen eines elektrischen Stroms an die Elektroden;
b) ein nicht ausdehnbarer Fluidbehälter (12) in dichter Fluidverbindung (13) mit der Gaserzeugungseinheit, welcher eine Arzneimittellösung (24) enthält und zum Empfangen von an den Elektroden erzeugtem Sauerstoff und Wasserstoff (29) unter Druck ausgelegt ist, wobei der Behälter (12) einen Auslaßdurchgang (25) aufweist; und
c) eine Verabreichungsvorrichtung (27), die mit dem Auslaßdurchgang (25) in dem Behälter (12) in Verbindung steht, zum Empfangen einer verdrängten Arzneimittellösung (24) aus dem Behälter als Reaktion auf den Druck, der durch in der Gaserzeugungseinheit (11) erzeugten Sauerstoff und Wasserstoff (29) ausgeübt wird, welche in den Behälter (12) eintreten und die Arzneimittellösung aus dem Behälter (12) über den Auslaßdurchgang (25) verdrängen, und zum Leiten der Lösung (24) in den Körper eines Patienten, wobei die Vorrichtung durch das Hydrogel (19) gekennzeichnet ist, welches ein elastisches, elektrisch leitendes, festes, in Wasser gequollenes, negativ geladenes Polymernetzwerk umfaßt, das Festigkeit und Steifigkeit besitzt, wobei das negativ geladene Polymernetzwerk ein Säurepolymer umfaßt, das aus synthetischen, halbsynthetischen oder natürlichen Monomeren besteht, die Carbon- oder Sulfonsäuregruppen enthalten.

2. Durch Gas angetriebene
Arzneimittelverabreichungsvorrichtung nach Anspruch 1, wobei das Hydrogel ein Säurepolymernetzwerk ist, das aus Monomeren besteht, die Sulfonsäuregruppen enthalten.

3. Durch Gas angetriebene
Arzneimittelverabreichungsvorrichtung nach Anspruch 2 wobei die Monomere aus der aus Styrolsulfonsäure und 2-Acrylamido-2-methylpropansulfonsäure bestehenden Gruppe ausgewählte Mitglieder sind.

4. Durch Gas angetriebene
Arzneimittelverabreichungsvorrichtung nach Anspruch 1, wobei das Hydrogel ein Säurepolymernetzwerk ist, das aus Monomeren besteht, die Carbonsäuregruppen enthalten.

5. Durch Gas angetriebene
Arzneimittelverabreichungsvorrichtung nach Anspruch 4, wobei die Monomere aus der aus Acrylsäure, Methacrylsäure und Maleinsäure bestehenden Gruppe ausgewählte Mitglieder sind.

6. Durch Gas angetriebene
Arzneimittelverabreichungsvorrichtung nach Anspruch 1, wobei das Hydrogel ein natürliches Polymernetzwerk ist, das aus Dextransulfatgelen, Proteingelen, Heparingelen und Kombinationen davon besteht.

7. Durch Gas angetriebene
Arzneimittelverabreichungsvorrichtung nach Anspruch 1, wobei das negativ geladene Polymerhydrogel dazu ausgelegt ist, an den Elektroden erzeugte Wasserstoff- und Sauerstoffgase aus dem Hydrogel abwandern und leicht von irgendeiner Oberfläche des Hydrogels in der Gaserzeugungseinheit in das Gasabteil des Fluidbehälters strömen zu lassen.

8. Durch Gas angetriebene
Arzneimittelverabreichungsvorrichtung nach Anspruch 7, wobei das Hydrogel in Form einer Scheibe vorliegt.

## Revendications

1. Dispositif d'administration de médicaments entraîné par un gaz (10) pour administrer un médicament liquide (24) à une vitesse prédéterminée, comprenant :
a) une unité de génération de gaz (11) pour produire de l'oxygène et de l'hydrogène gazeux (29) par électrolyse d'eau, comprenant un boîtier (14) auquel est collé un hydrogel (19), des électrodes (20, 21) insérées dans ledit hydrogel et s'étendant vers l'extérieur dudit boîtier et des moyens fixés auxdites électrodes vers l'extérieur dudit boîtier pour appliquer un courant électrique auxdites électrodes ;
b) un réservoir de fluide non extensible (12) en communication fluidique étanche (13) avec ladite unité de génération de gaz, contenant une solution médicamenteuse (24) et configuré pour recevoir l'oxygène et l'hydrogène (29) produits au niveau desdites électrodes sous pression, ledit réservoir (12) ayant un passage d'évacuation (25) ; et
c) des moyens d'administration (27) en communication avec ledit passage d'évacuation (25) dans ledit réservoir (12) pour recevoir ladite solution médicamenteuse déplacée (24) depuis ledit réservoir en réponse à la pression exercée par l'oxygène et l'hydrogène (29) générés dans ladite unité de génération de gaz (11) entrant dans ledit réservoir (12) et déplacer la solution médicamenteuse dudit réservoir (12), à travers ledit passage d'évacuation (25) et diriger ladite solution (24) dans le corps d'un patient, ledit dispositif étant caractérisé en ce que ledit hydrogel (19) comprend un réseau polymère chargé négativement, gonflé d'eau, solide, électroconducteur et élastique possédant de la résistance et de la rigidité, dans lequel ledit réseau polymère chargé négativement comprend un polymère acide composé de monomères synthétiques, semi-synthétiques ou naturels qui contiennent des groupes acide carboxylique ou sulfonique.

2. Dispositif d'administration de médicaments entraîné par un gaz selon la revendication 1, dans lequel l'hydrogel est un réseau polymère acide composé de monomères qui contiennent des groupes acide sulfonique.

3. Dispositif d'administration de médicaments entraîné par un gaz selon la revendication 2, dans lequel lesdits monomères sont des éléments choisis dans le groupe composé de l'acide sulfonique de styrène et l'acide sulfonique de 2-acrylamido-2-méthylpropane.

4. Dispositif d'administration de médicaments entraîné par un gaz selon la revendication 1, dans lequel l'hydrogel est un réseau polymère acide composé de monomères qui contiennent des groupes acide carboxylique.

5. Dispositif d'administration de médicaments entraîné par un gaz selon la revendication 4, dans lequel lesdits monomères sont des éléments choisis dans le groupe composé de l'acide acrylique, l'acide méthacrylique et l'acide maléique.

6. Dispositif d'administration de médicaments entraîné par un gaz selon la revendication 1, dans lequel l'hydrogel est un réseau polymère naturel composé de gels de sulfate de dextrane, de gels protéiniques, de gels d'héparine, et de leurs combinaisons.

7. Dispositif d'administration de médicament entraîné par un gaz selon la revendication 1, dans lequel ledit hydrogel polymère chargé négativement est configuré de manière à permettre à l'hydrogène et à l'oxygène gazeux produits dans les électrodes de migrer hors dudit hydrogel et de passer facilement de n'importe quelle surface dudit hydrogel dans ladite unité de génération de gaz audit compartiment à gaz dudit récipient de fluide.

8. Dispositif d'administration de médicament entraîné par un gaz selon la revendication 7, dans lequel ledit hydrogel se présente sous la forme d'un disque.
